(19) Europäisches Patentamt
European Patent Office
Office européen des brevets

(11) Veröffentlichungsnummer: **0 327 976 A2**

# (12) EUROPÄISCHE PATENTANMELDUNG

(21) Anmeldenummer: **89101840.0**

(22) Anmeldetag: **03.02.89**

(51) Int. Cl.4: **G03C 7/26 , G03C 7/36 , //C07C143/82**

(30) Priorität: **06.02.88 DE 3803664**

(43) Veröffentlichungstag der Anmeldung: **16.08.89 Patentblatt 89/33**

(84) Benannte Vertragsstaaten: **BE DE FR GB NL**

(71) Anmelder: **Agfa-Gevaert AG**

**D-5090 Leverkusen 1(DE)**

(72) Erfinder: **Helling, Günter, Dr.**
**In der Hildscheid 16**
**D-5068 Odenthal(DE)**
Erfinder: **Himmelmann, Wolfgang, Dr.**
**Im Ziegelfeld 7**
**D-5090 Leverkusen 3(DE)**
Erfinder: **Kunitz, Friedrich-Wilhelm, Dr.**
**Walter-Flex-Strasse 20**
**D-5090 Leverkusen 1(DE)**
Erfinder: **Mäder, Helmut, Dr.**
**Theodor-Storm-Weg 1**
**D-5068 Odenthal(DE)**
Erfinder: **Mücke, Bruno, Dr.**
**Alte Wipperfürther Strasse 105**
**D-5060 Bergisch Gladbach 2(DE)**
Erfinder: **Voigt, Armin, Dr.**
**An der Ruthen 6**
**D-5000 Köln 80(DE)**

## (54) Farbfotografisches Aufzeichnungsmaterial zur Herstellung farbiger Aufzeichnungsbilder.

(57) Farbfotografisches Aufzeichnungsmaterial mit einem lichtreflektierenden Schichtträger und mindestens drei darauf angeordneten lichtempfindlichen Silberhalogenidemulsionsschichten unterschiedlicher Spektralempfindlichkeit, denen ein Blaugrünkuppler, ein Purpurkuppler und ein Gelbkuppler jeweils spektral zugeordnet ist, wobei die Silberhalogenidschichten einen Chloridgehalt von > 95 Mol-% aufweisen und der blauempfindlichen Silberhalogenidemulsionsschicht ein nicht diffundierender α-Acylacetanilid-Gelbkuppler der folgenden Formel (I) zugeordnet ist:

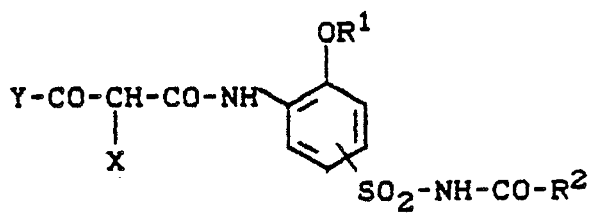

(I)

worin
X, Y, $R^1$ und $R^2$ die in der Beschreibung angegebene Bedeutung haben,
sowie die Schichten des Aufzeichnungsmaterials mit einem Härtungsmittel der folgenden Formel (II) gehärtet sind

EP 0 327 976 A2

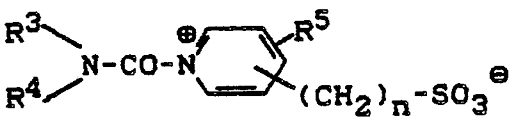

(II)

worin

$R^3$, $R^4$, $R^5$ und n die in der Beschreibung angegebene Bedeutung haben,

zeigt in Verbindung mit einem Schnellverarbeitungsprozeß einen geringen Gelbschleier ($D_{min}$) bei gleichzeitig neutraler Farbwiedergabe.

## Farbfotografisches Aufzeichnungsmaterial zur Herstellung farbiger Aufsichtsbilder

Die Erfindung betrifft ein lichtempfindliches farbfotografisches Aufzeichnungsmaterial mit einem lichtreflektierenden Schichtträger und mindestens drei darauf angeordneten lichtempfindlichen Silberhalogenidemulsionsschichten unterschiedlicher Spektralempfindlichkeit, denen ein Blaugrünkuppler, ein Purpurkuppler und ein Gelbkuppler jeweils spektral zugeordnet ist. Durch Auswahl spezieller, schnellentwickelbarer Silberhalogenidemulsionen, Gelbkuppler und Härtungsmittel wird ein Aufzeichnungsmaterial erhalten, welches in einem Schnellverarbeitungsprozeß farbneutrale Bilder mit geringem Schleier ($D_{min}$) des gelben Teilfarbenbildes liefert.

Es ist bekannt, farbige fotografische Bilder durch chromogene Entwicklung herzustellen, d. h. dadurch, daß man bildmäßig belichtete Silberhalogenidemulsionsschichten in Gegenwart geeigneter Farbkuppler mittels geeigneter farbbildender Entwicklersubstanzen - sogenannter Farbentwickler - entwickelt, wobei das in Übereinstimmung mit dem Silberbild entstehende Oxidationsprodukt der Entwicklersubstanzen mit dem Farbkuppler unter Bildung eines Farbstoffbildes reagiert. Als Farbentwickler werden gewöhnlich aromatische, primäre Aminogruppen enthaltende Verbindungen, insbesondere solche vom p-Phenylendiamintyp, verwendet.

Eine wichtige Voraussetzung für die Schnellverarbeitung von farbfotografischen Aufzeichnungsmaterialien ist die Verwendung von schnellentwickelbaren Emulsionen. In dieser Hinsicht haben sich chloridreiche Silberhalogenidemulsionen als vorteilhaft erwiesen. Derartige Emulsionen werden beispielsweise in US-A-4 269 927 und WO 87/04535 beschrieben. Geeignet sind Emulsionen mit mindestens 80 Mol-%, vorzugsweise mit 95 - 100 Mol-% Chloridanteil.

An die Farbkuppler, sowie an die daraus durch chromogene Entwicklung erhaltenen Farbstoffe werden insbesondere hinsichtlich der Schnellentwicklung eine Reihe von Forderungen gestellt. So soll die Kupplungsgeschwindigkeit der Farbkuppler mit dem Oxidationsprodukt des Farbentwicklers möglichst groß sein und es soll eine möglichst hohe maximale Farbdichte erzielt werden können. Die Farbkuppler sowie die daraus erhaltenen Farbstoffe müssen hinreichend stabil sein gegenüber Licht, erhöhter Temperatur und Feuchtigkeit. Dies gilt sowohl für frisches Material, als auch für verarbeitetes Material.

Beispielsweise darf der in den Bildweißen des verarbeiteten Materials noch vorhandene restliche Kuppler nicht vergilben. Außerdem sollten die Farbstoffe hinreichend beständig sein gegenüber gasförmigen reduzierenden oder oxidierenden Agentien. Sie müssen ferner diffusionsfest in der Bildschicht verankert sein und sollen sich bei der chromogenen Entwicklung als möglichst feines Korn abscheiden. Schließlich müssen die aus den Farbkupplern bei der chromogenen Entwicklung entstehenden Farbstoffe eine günstige Absorptionskurve aufweisen mit einem Maximum, das der Farbe des jeweils gewünschten Teilbildes entspricht, und möglichst geringen Nebenabsorptionen.

Die bekannten Gelbkuppler erfüllen jedoch die an sie gerichtete Anforderung nicht in jeder Hinsicht. Ein besonderes Problem wird zur Zeit darin gesehen, daß in manchen Verarbeitungsgängen die Anwesenheit von Benzylalkohol unabdingbar ist um gleichmäßige hohe Farbdichten insbesondere der Gelbfarbstoffe zu erzielen. Die Anwesenheit von Benzylalkohol im Entwickler gibt jedoch leicht Anlaß zur Abscheidung von teerartigen Massen im Entwicklertank. Ein weiterer Nachteil beruht auf der leichten Oxidierbarkeit des Benzylalkohols, was eine sorgfältige Überwachung und Konstanthaltung des Entwicklerbades erfordert um gleichmäßige Entwicklungsergebnisse zu gewährleisten. Es ist daher erwünscht, derartige Aufzeichnungsmaterialien in Abwesenheit von Benzylalkohol zu entwickeln. Bei einem Schnellverarbeitungsprozeß müssen somit Gelbkuppler verwendet werden, die auch in benzylalkoholfreien Entwicklungsbädern ausreichende Kupplungsgeschwindigkeit zeigen und somit für die nötige Maximaldichte sorgen. Diese Eigenschaften erfüllen α-Acylacetanilid-Gelbkuppler, deren Anilidgruppe mit einer N-Acylsulfamoylgruppe substituiert sind.

Die Neigung zur Ausbildung von Gelbschleier ist bei der Verwendung von chloridreichen Silberhalogenidemulsionen in Verbindung mit Gelbkupplern des oben beschriebenen Typs bei einer Schnellverarbeitung besonders ausgeprägt.

Der Erfindung liegt die Aufgabe zugrunde, ein Silberhalogenid und Farbkuppler enthaltendes farbfotografisches Aufzeichnungsmaterial zu erstellen, mit dem unter Verwendung von chloridreichen Emulsionen und Gelbkuppler hoher Kupplungsgeschwindigkeit durch chromogene Entwicklung farbiger Aufsichtsbilder mit möglichst geringem Schleier ($D_{min}$) des gelben Teilfarbenbildes hergestellt werden können. Es wurde nun gefunden, daß unter Verwendung von carboxylgruppen-aktivierenden Härtungsmitteln ein Teilfarbenbild mit sehr geringem Schleier erhalten wird.

Gegenstand der Erfindung ist ein farbfotografisches Aufzeichnungsmaterial, das auf einem reflektierenden Schichtträger mindestens eine blauempfindliche Silberhalogenidemulsionsschicht und einen dieser zugeordneten Gelbkuppler, mindestens eine grünempfindliche Silberhalogenidemulsionsschicht und einen

dieser zugeordneten Purpurkuppler, mindestens eine rotempfindliche Silberhalogenidemulsionsschicht und einen dieser zugeordneten Blaugrünkuppler und gegebenenfalls weitere nicht lichtempfindliche Schichten enthält, dadurch gekennzeichnet, daß

a) die Silberhalogenidemulsionsschichten einen Chloridgehalt von ≥ 95 Mol % aufweisen

b) der blauempfindlichen Silberhalogenidemulsionsschicht ein nicht diffundierender α-Acylacetanilid-Gelbkuppler der folgenden Formel I zugeordnet ist,

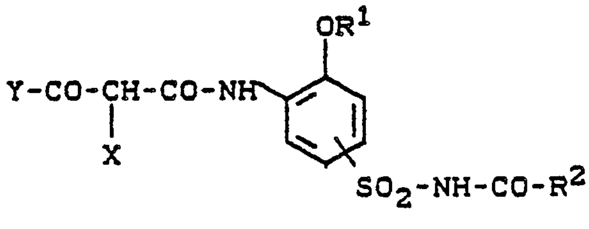

(I)

worin bedeuten:

Y einen aliphatischen oder cycloaliphatischen Rest;

X ein Wasserstoffatom oder eine bei Farbkupplung abspaltbare Gruppe;

$R^1$ einen Alkylrest mit 12 bis 20 C-Atomen oder eine Bindung an ein Polymergerüst;

$R^2$ einen Alkylrest mit 1 bis 4 C-Atomen oder eine Bindung an ein Polymergerüst;

c) die Schichten des Aufzeichnungsmaterials mit einem Härtungsmittel der folgenden Formel II gehärtet sind

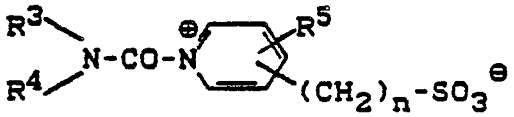

(II)

worin bedeuten:

$R^3$ und $R^4$ einzeln gleich oder verschieden, jeweils eine Alkylgruppe mit 1 bis 8 Kohlenstoffatomen oder eine gegebenenfalls mit einer Alkylgruppe mit 1 oder 2 Kohlenstoffatomen oder mit einem Halogenatom substituierte Aryl- oder Aralkylgruppe, oder zusammen die zur Vervollständigung eines gegebenenfalls mit einer Alkylgruppe mit 1 oder 2 Kohlenstoffatomen oder mit einem Halogenatom substituierten heterocyclischen Ringes, z. B. eines Piperidin- oder Morpholinringes erforderlichen Atome,

$R^5$ ein Wasserstoffatom oder eine Alkylgruppe mit 1 oder 2 Kohlenstoffatomen,

n gleich 0 oder 2.

Ein in Formel (I) durch Y dargestellter aliphatischer Rest ist bevorzugt ein tert.-Alkylrest, insbesondere tert.-Butyl. Ein durch Y dargestellter cycloaliphatischer Rest ist beispielsweise ein Cyclohexyl-, Norbornyl- oder Adamantylrest.

Eine durch X dargestellte bei Farbkupplung abspaltbare Gruppe ist bevorzugt eine über ein Sauerstoffatom oder über ein Stickstoffatom, insbesondere ein Ringstickstoffatom angeknüpfte cyclische Gruppe, z. B. ein über ein Ringstickstoffatom angeknüpfter, gegebenenfalls substituierter 5- oder 6-gliedriger heterocyclischer Ring. Solche auch als Fluchtgruppen bezeichnete abspaltbare Gruppen verleihen üblicherweise dem Kuppler das Verhalten eines 2-Äquivalentkupplers, d. h. der Kuppler benötigt zur Farbkupplung nur halbsoviel entwickelbares Silberhalogenid, wie der entsprechende 4-Äquivalentkuppler, bei dem X ein Wasserstoffatom bedeutet. Beispiele für geeignete Fluchtgruppen sind im folgenden angegeben.

Fluchtgruppen

1. 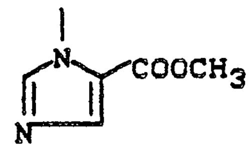

2. 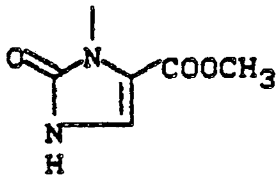

3. 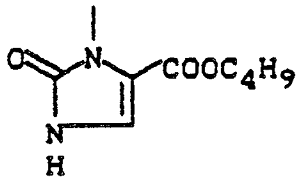

Fluchtgruppen (Fortsetzung)

4. 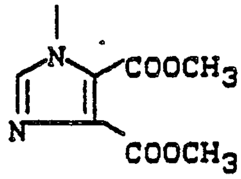

5. 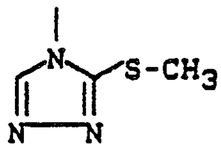

6. 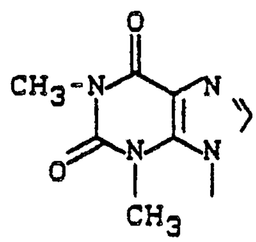

7. 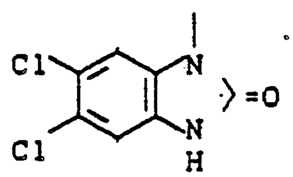

8. 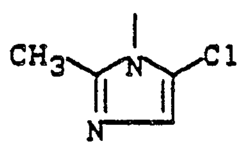

9. 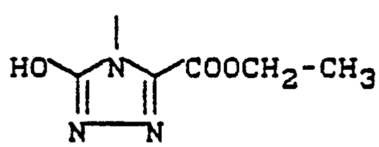

Fluchtgruppen (Fortsetzung)

10. 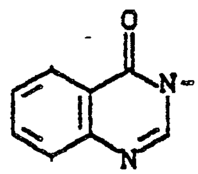

11. 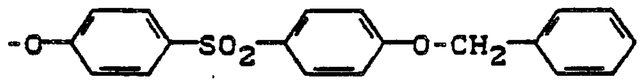

12. 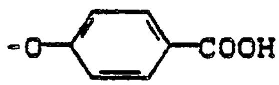

13. 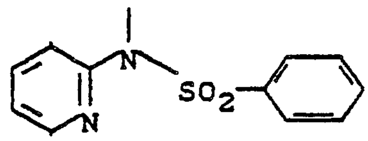

14. 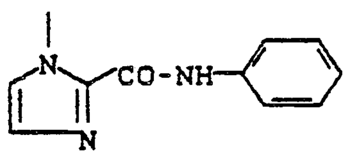

15. 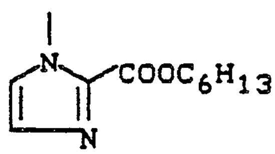

16. 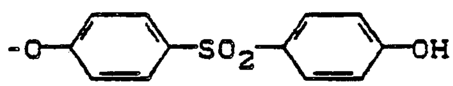

17. 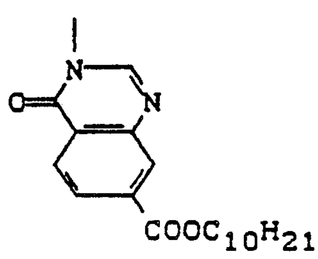

18. 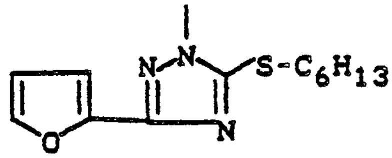

$R^1$ und $R^2$ kann ein polymerisierter Rest einer ethylenisch polymerisierbaren Gruppe der Formel

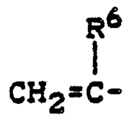 oder 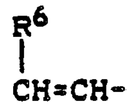

sein, worin
$R^6$ H, Halogen, insbesondere Chlor, -COOH oder Alkyl, vorzugsweise mib 1 bis 4 C-Atomen und gegebenenfalls mit -COOH substituiert, bedeuten.

Die ethylenisch ungesättigte Gruppe kann direkt oder indirekt über ein Bindeglied L gebunden sein. Ein solches Bindeglied -L- kann eine zusammengesetzte Struktur aufweisen und beispielsweise wie folgt dargestellt werden:

$-(L^0)_k-(L^1)_l-(L^2)_m-(L^3)_n-(L^4)_o-(L^5)_p-(L^6)_q-(L^7)_r-$

worin $L^0$ den dem heterocyclischen Ring und $L^7$ den der ethylenisch ungesättigten Gruppe zunächst gelegenen Teil des Bindegliedes bezeichnen und worin bedeuten (gleich oder verschieden):

$L^0$, $L^2$, $L^4$, $L^6$ -O-, -NR-, -NRCO-, -CONR-, $-NRSO_2-$, $-SO_2NR-$, -COO-, -O-CO-, -NR-CO-NR-, -O-CO-NR-, -NR-COO- (mit R = H oder Alkyl, das gegebenenfalls mit Carboxyl substituiert sein kann);

$L^1$, $L^3$, $L^5$, $L^7$ Alkylen, Aralkylen, Arylen, gegebenenfalls mit Carboxy substituiert;

k,l,m,n,o,p,q,r jeweils 0 oder 1, wobei gilt, daß

$1 - m + n - o + p - q = 0$.

Eine durch $L^1$, $L^3$, $L^5$, $L^7$ dargestellte Alkylengruppe kann geradkettig oder verzweigt sein und bis zu 20 C-Atomen aufweisen und gegebenenfalls mit Carboxyl substituiert sein.

Eine durch $L^1$, $L^3$, $L^5$ dargestellte Aralkylengruppe ist beispielsweise eine der folgenden Gruppen:

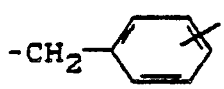 , 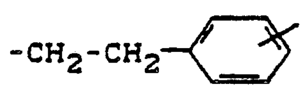 , 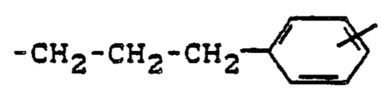

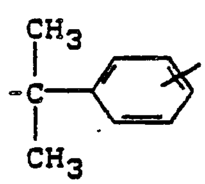 , 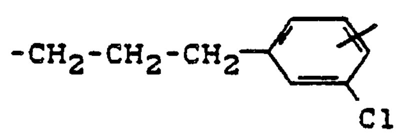 , 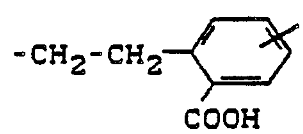

Eine durch $L^1$, $L^3$, $L^5$, $L^7$ dargestellte Arylengruppe ist vorzugsweise eine Phenylengruppe, die substituiert sein kann, z.B. mit Alkyl, Alkoxy, Halogen, Acylamino, Carboxyl oder einem Substituenten, der eine Carboxylgruppe enthält.

Einige Beispiele der in dem erfindungsgemäßen Material verwendeten Gelbkuppler sind im folgenden aufgeführt:

Y-1 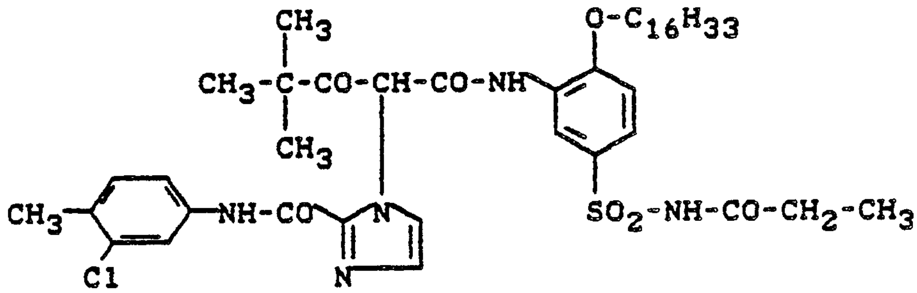

Y-2 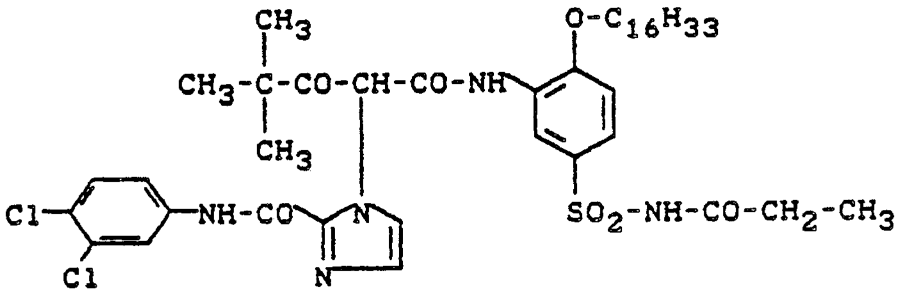

Y-3 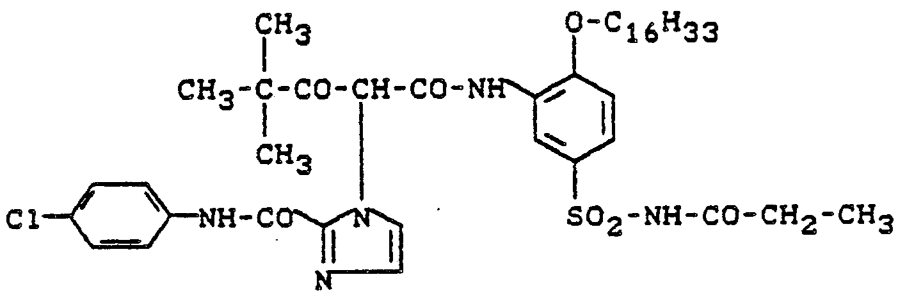

Y-4

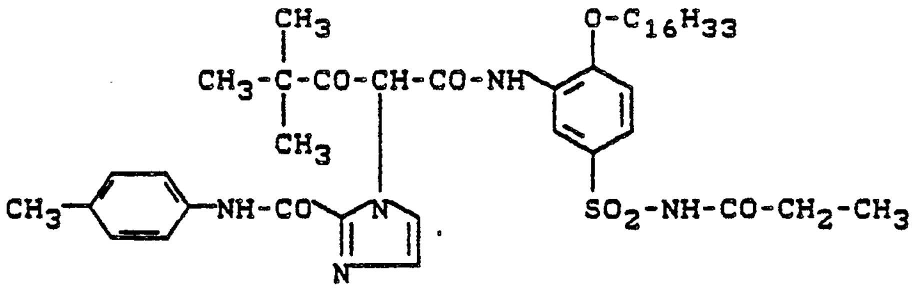

Y-5

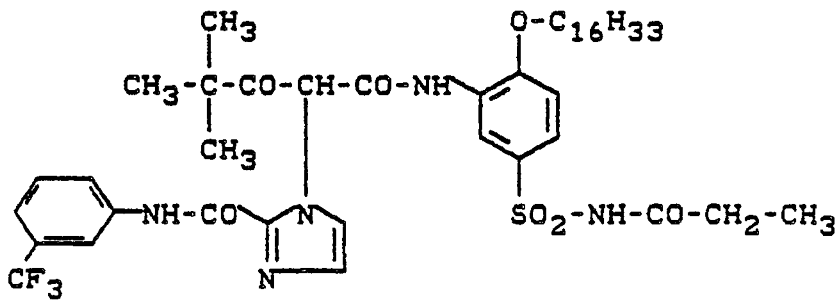

Y-6

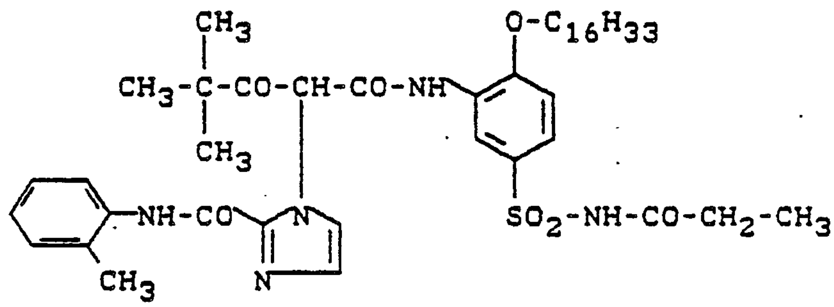

Y-7

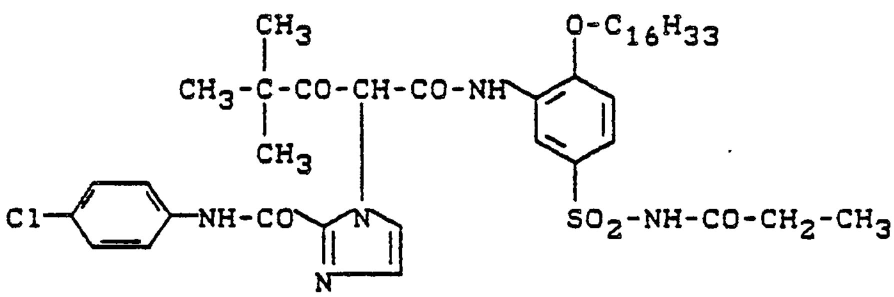

Y-8

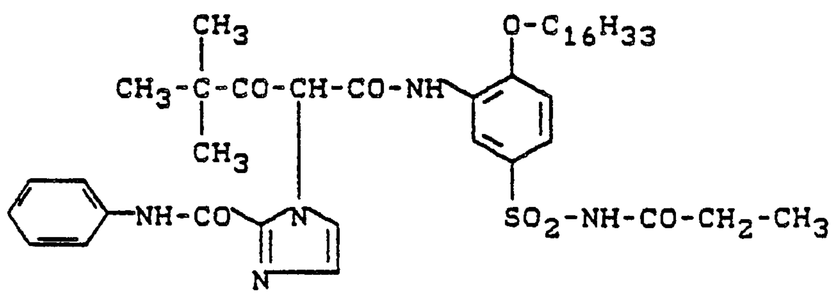

Y-9

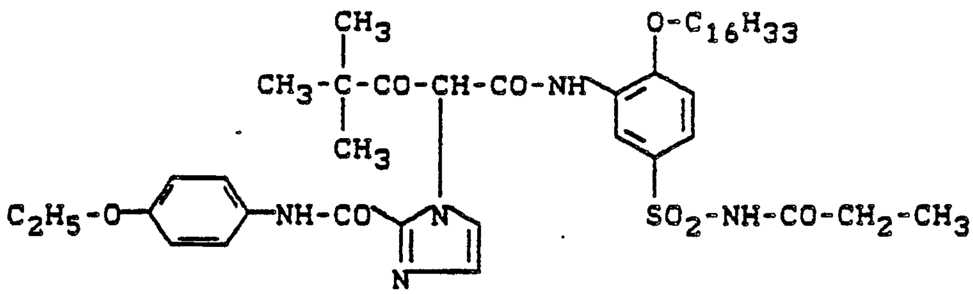

Y-10

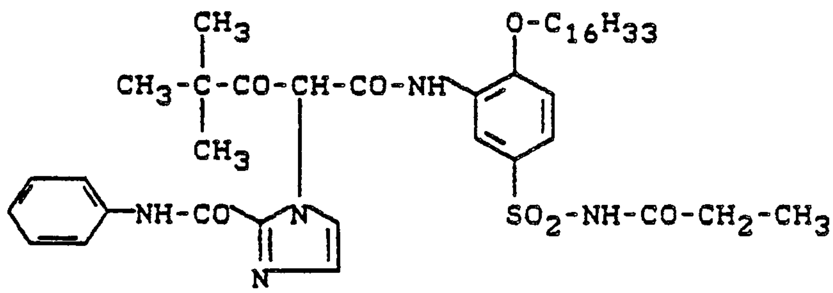

Y-11

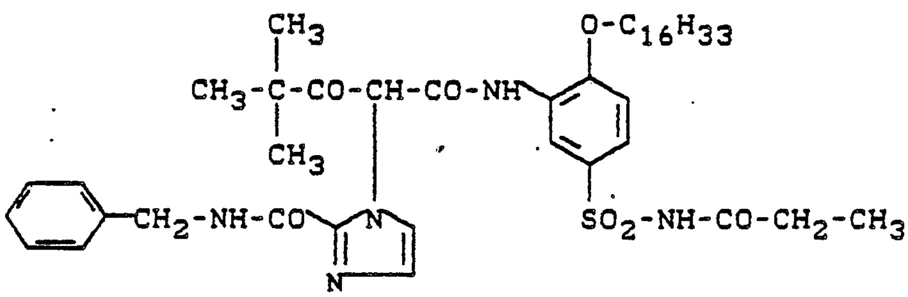

Y-12

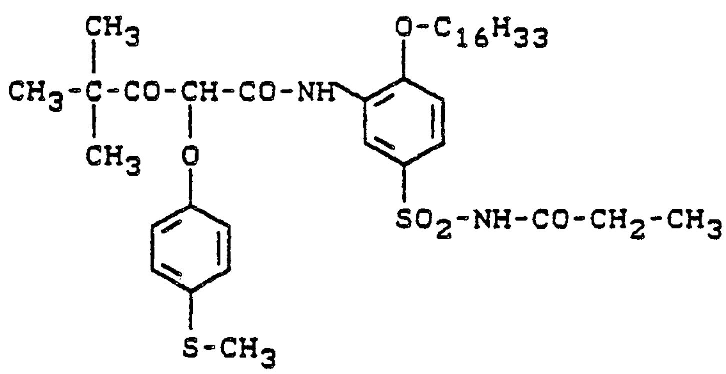

Y-13 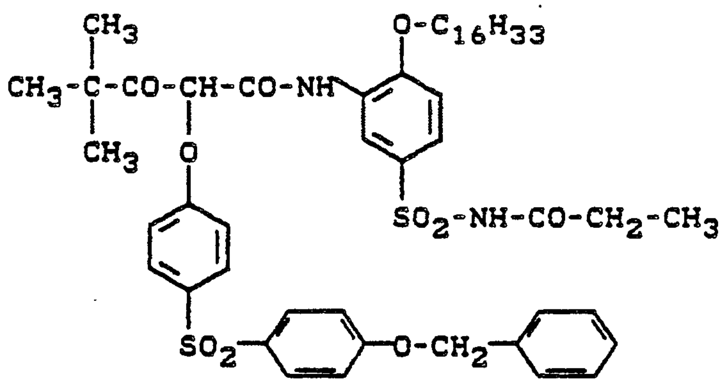

Y-14 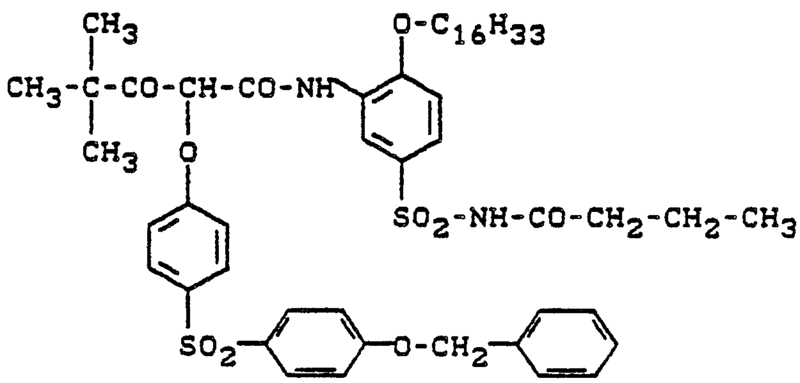

Y-15 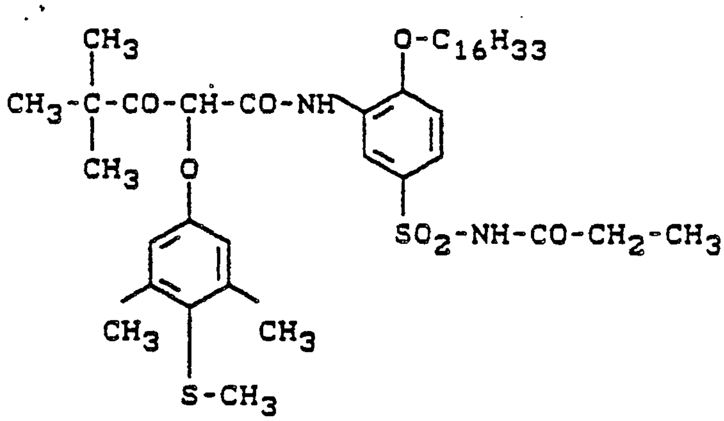

Y-16

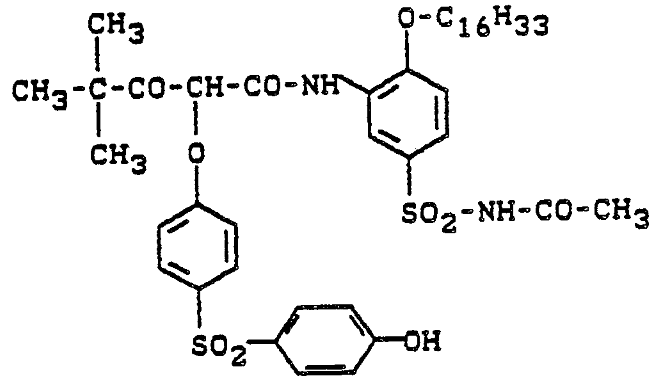

Y-17

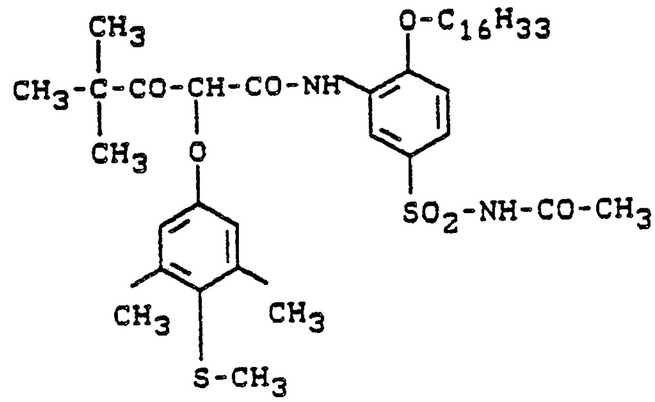

Y-18

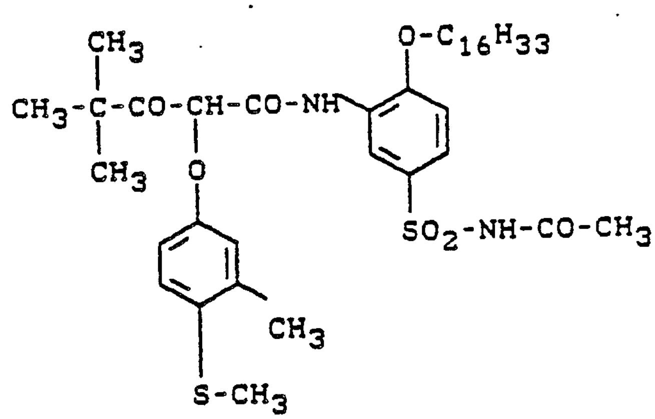

Y-19 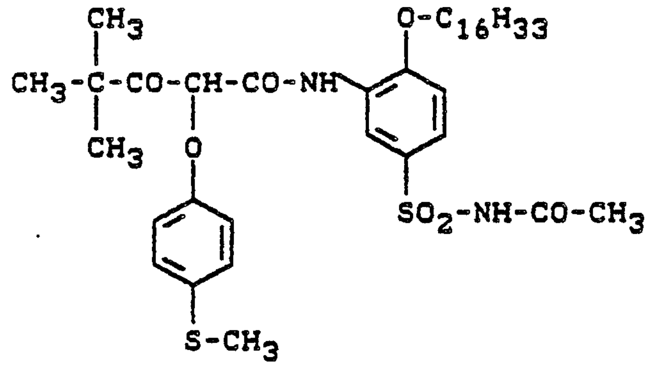

Y-20 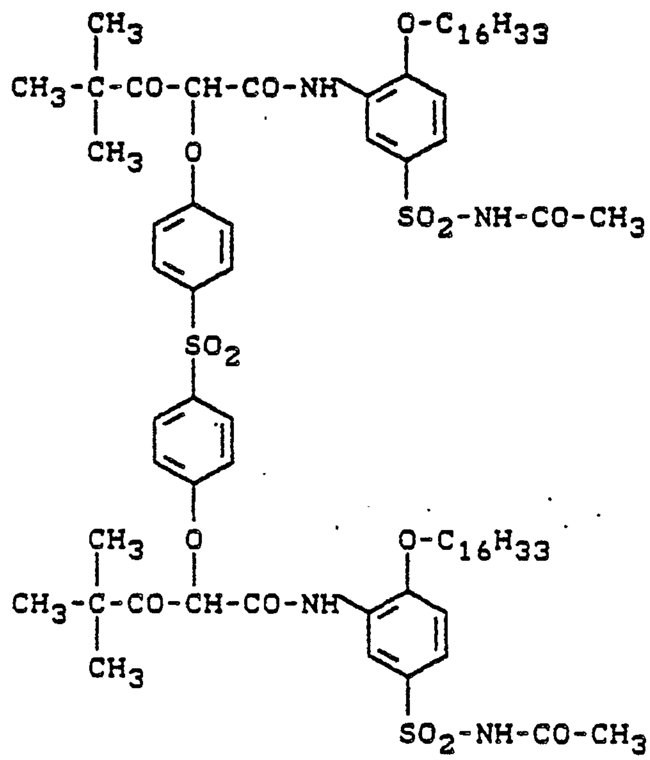

Y-21 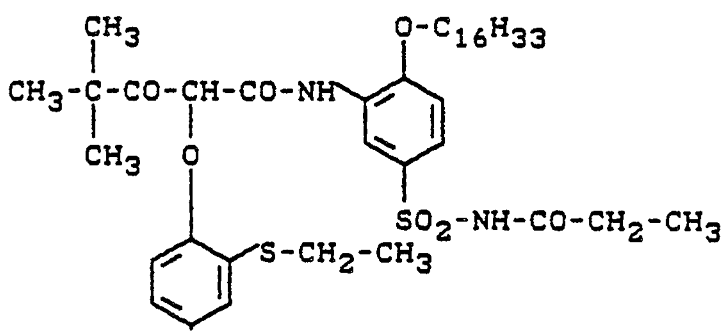

Y-22

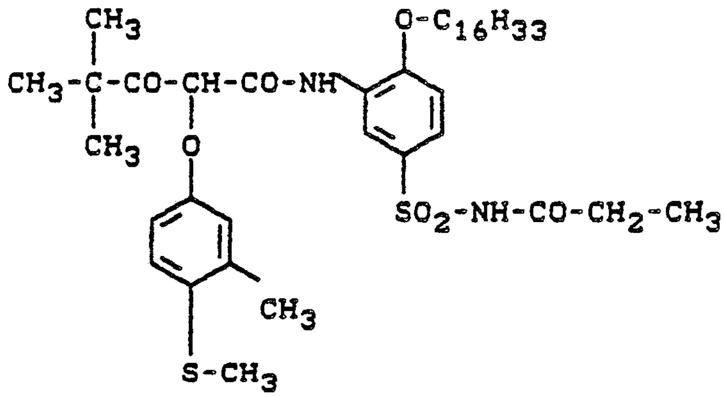

Y-23

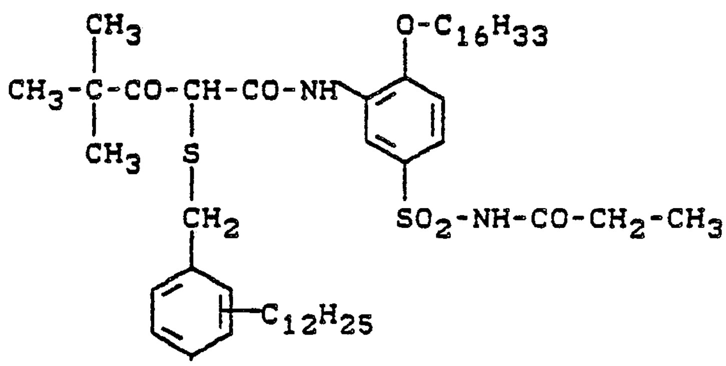

Y-24

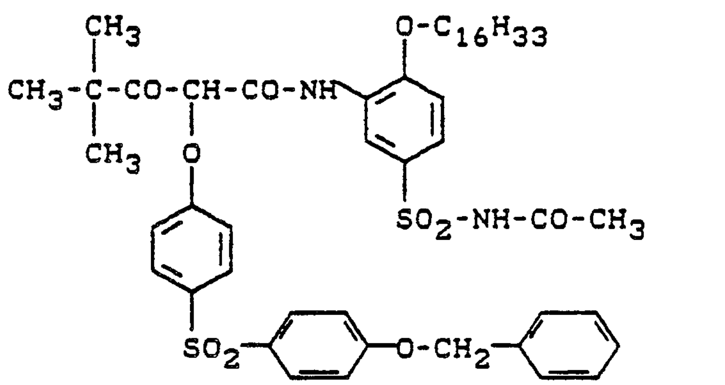

Y-25

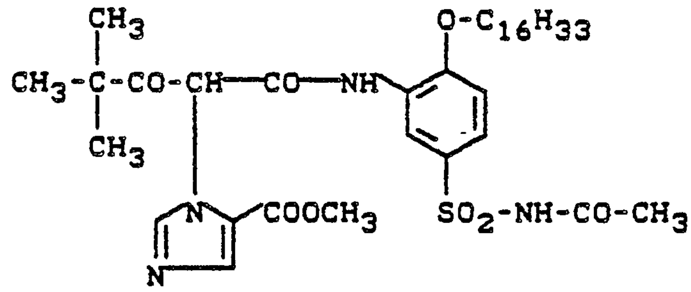

Y-26

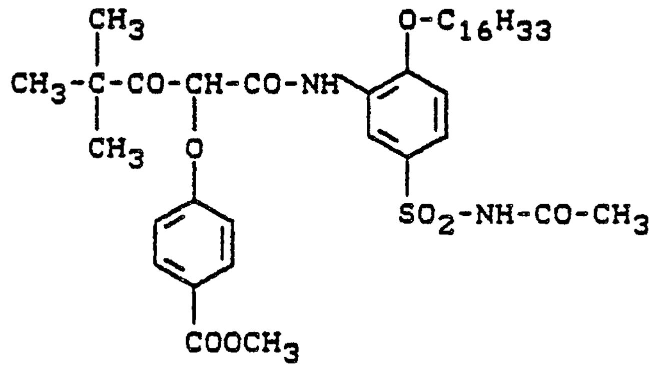

Y-27

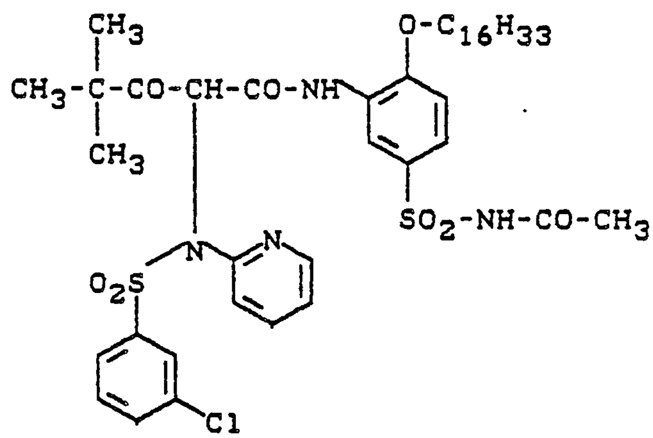

Y-28

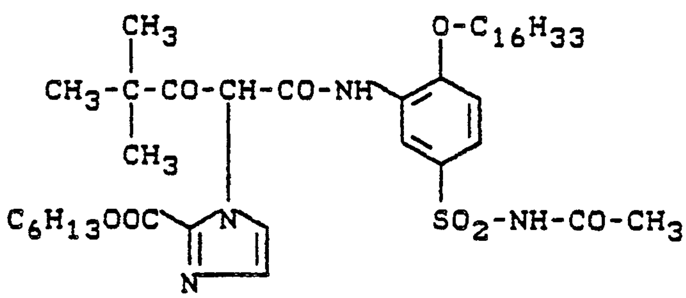

Y-29

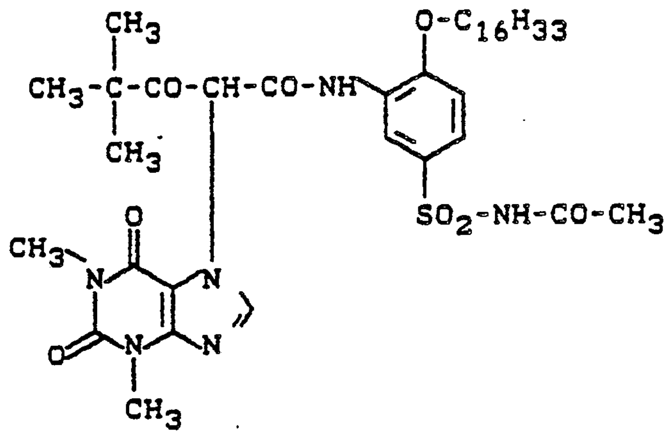

Y-30

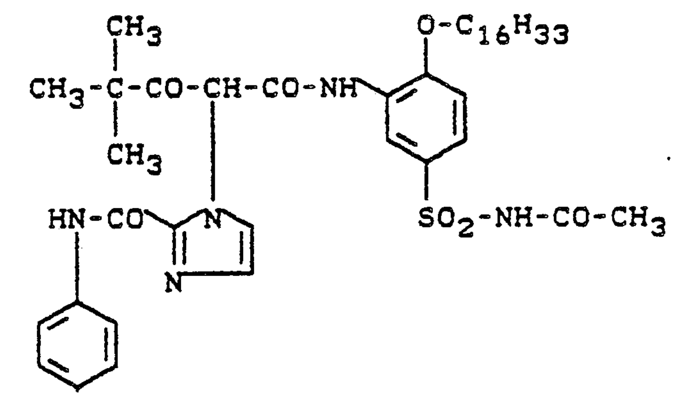

Y-31

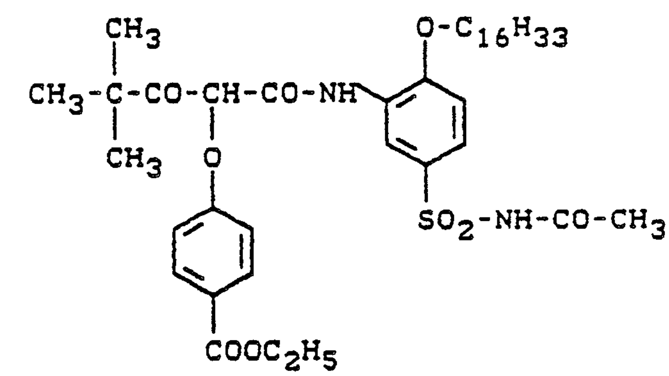

Y-32

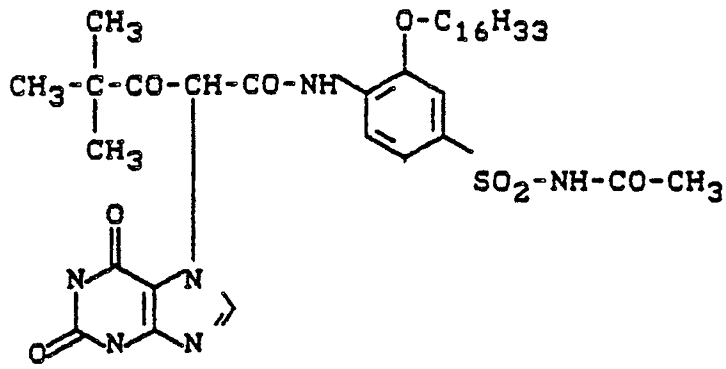

Y-33

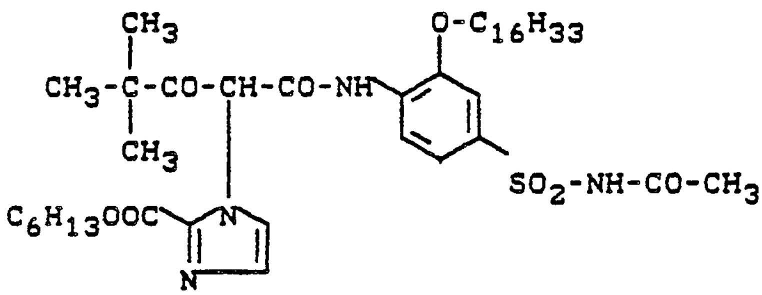

Y-34

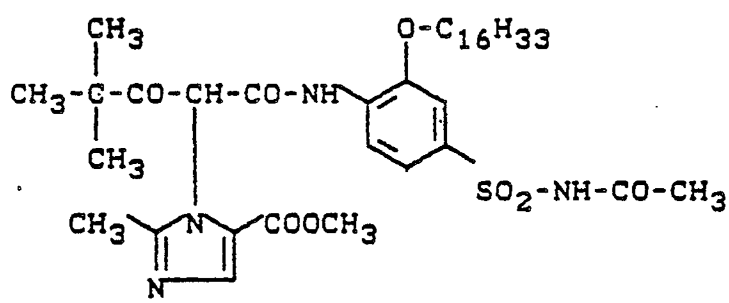

Y-35

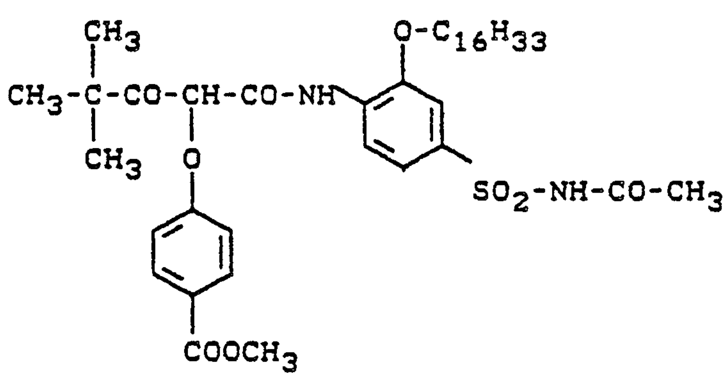

Y-36

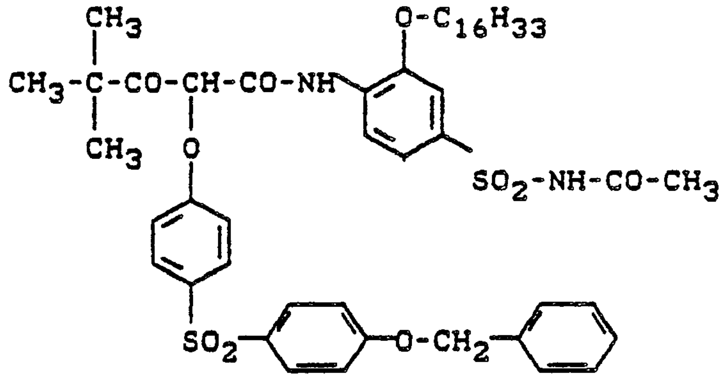

Y-37

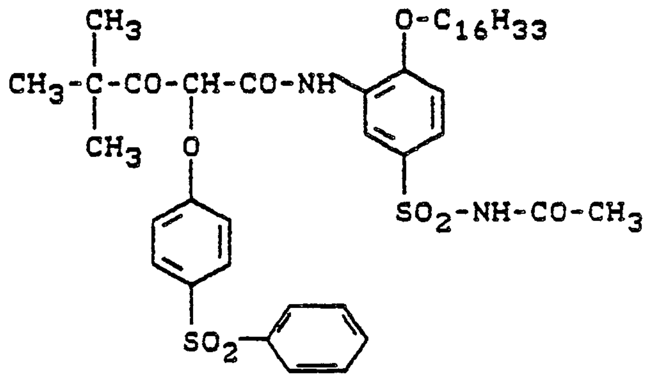

Y-38

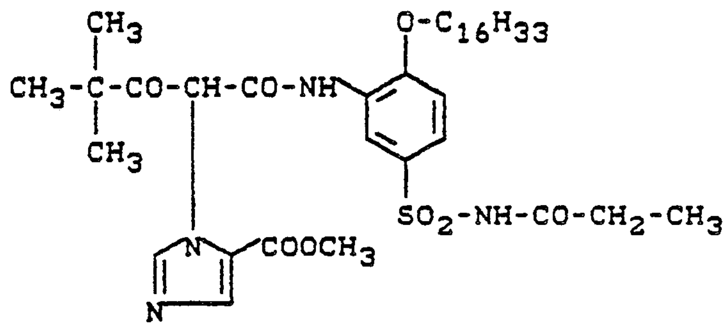

Y-39

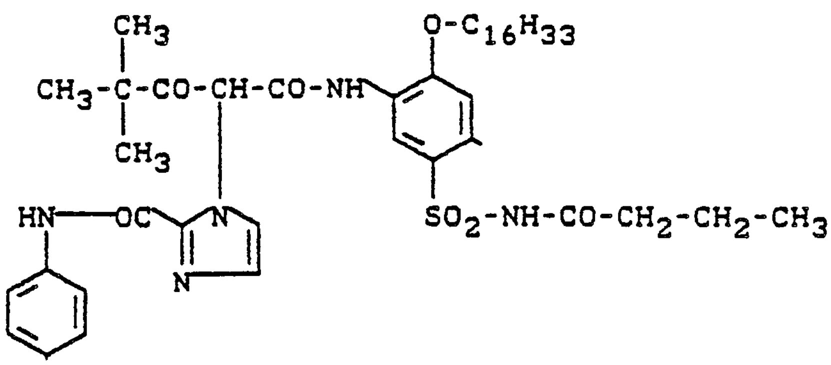

Y-40

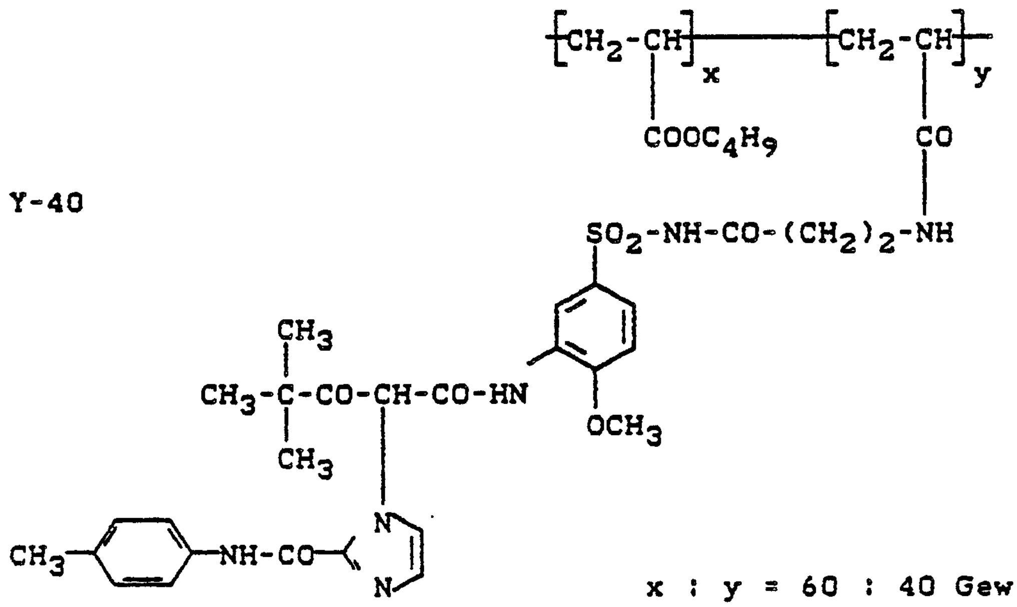

x : y = 60 : 40 Gew.-%

Die zur Schnellentwicklung geeigneten Silberhalogenidemulsionen enthalten mindestens 95 Mol-% Chlorid, wobei der zu 100 Mol-% ergänzende Rest aus 0 bis 5 Mol-% Bromid, Iodid und Rhodanid, einzeln oder in Kombination, besteht. Rhodanid wird hierbei als Halogenidersatz angesehen (Pseudohalogenid). Diese Halogenide und Pseudohalogenide werden vorzugsweise in folgenden Mengen eingesetzt: 0,01 bis 0,5 Mol-% Iodid, 0,02 bis 5 Mol-% Bromid und 0,02 bis 5 Mol-% Rhodanid. Die Korngröße variiert nach Schicht von 0,3 bis 0,9 µm.

Es kann sich um überwiegend kompakte Kristalle handeln, die z.B. regulär kubisch oder oktaedrisch sind oder Übergangsformen aufweisen können. Vorzugsweise können aber auch plättchenförmige Kristalle vorliegen, deren durchschnittliches Verhältnis von Durchmesser zu Dicke größer als 5:1, vorzugsweise größer als 8:1, ist, wobei der Durchmesser eines Kornes definiert ist als der Durchmesser eines Kreises mit einem Kreisinhalt entsprechend der projizierten Fläche des Kornes.

Die Silberhalogenidkörner können auch einen mehrfach geschichteten Kornaufbau aufweisen, im einfachsten Fall mit einem inneren und einem äußeren Kornbereich (core/shell), wobei die Halogenidzusammensetzung und/oder sonstige Modifizierungen, wie z.B. Dotierungen der einzelnen Kornbereiche unterschiedlich sind.

Verwiesen wird zum Beispiel auf GB-1.027.146. Dort sind Silberhalogenide mit geschichtetem Kornaufbau und Methoden zu ihrer Herstellung angegeben. Im vorliegenden Fall werden auf die Silberchloridkörner andere Silberhalogenide aufgefällt. Bevorzugt ist Silberbromid, das gegebenenfalls geringe Mengen Silberiodid enthalten kann. Die Menge der aufgefällten anderen Silberhalogenide beträgt, wie weiter oben angegeben, bis zu 5 Mol-%. Die für den jeweiligen Fall optimale molare Menge des anderen Silberhalogenids kann durch einfache Versuche festgestellt werden.

Die mittlere Korngröße der Emulsionen liegt vorzugsweise zwischen 0,2 µm und 1,0 µm. Die Korngrößenverteilung ist vorzugsweise homodispers. Homodisperse Korngrößenverteilung bedeutet, daß 95 % der Körner nicht mehr als ± 30% von der mittleren Korngröße abweichen. Die Emulsionen können außer dem Silberhalogenid auch organische Silbersalze enthalten, z.B. Silberbenztriazolat oder Silberbehenat.

Es können zwei oder mehrere Arten von Silberhalogenidemulsionen, die getrennt hergestellt werden, als Mischung verwendet werden.

Die fotografischen Emulsionen können naoh verschiedenen Methoden (z.B. P. Glafkides, Chimie et Physique Photographique, Paul Montel, Paris (1967), G.F. Duffin, Photographic Emulsion Chemistry, The Focal Press, London (1966), V.L. Zelikman et al, Making and Coating Photographic Emulsion, The Focal Press, London (1966) aus löslichen Silbersalzen und löslichen Halogeniden hergestellt werden.

Die Fällung des Silberhalogenids erfolgt bevorzugt in Gegenwart des Bindemittels, z.B. der Gelatine und kann im sauren, neutralen oder alkalischen pH-Bereich durchgeführt werden, wobei vorzugsweise Silberhalogenidkomplexbildner zusätzlich verwendet werden. Zu letzteren gehören z.B. Ammoniak, Thioether, Imidazol, Ammoniumthiocyanat oder überschüssiges Halogenid. Die Zusammenführung der wasserlöslichen Silbersalze und der Halogenide erfolgt wahlweise nacheinander nach dem single-jet- oder gleichzeitig nach dem double-jet-Verfahren oder nach beliebiger Kombination beider Verfahren. Bevorzugt

wird die Dosierung mit steigenden Zuflußraten, wobei die "kritische" Zufuhrgeschwindigkeit, bei der gerade noch keine Neukeime entstehen, nicht überschritten werden sollte. Der pAg-Bereich kann während der Fällung in weiten Grenzen variieren, vorzugsweise wird das sogenannte pAg-gesteuerte Verfahren benutzt, bei dem ein bestimmter pAg-Wert konstant gehalten oder ein definiertes pAg-Profil während der Fällung durchfahren wird. Neben der bevorzugten Fällung bei Halogenidüberschuß ist aber auch die sogenannte inverse Fällung bei Silberionenüberschluß möglich. Außer durch Fällung können die Silberhalogenidkristalle auch durch physikalische Reifung (Ostwaldreifung), in Gegenwart von überschüssigem Halogenid und/oder Silberhalogenidkomplexierungsmittel wachsen. Das Wachstum der Emulsionskörner kann sogar überwiegend durch Ostwaldreifung erfolgen, wobei vorzugsweise eine feinkörnige, sogenannte Lippmann-Emulsion, mit einer schwerer löslichen Emulsion gemischt und auf letzterer umgelöst wird.

Während der Fällung und/oder der physikalischen Reifung der Silberhalogenidkörner können auch Salze oder Komplexe von Metallen, wie Cd, Zn,Pb, Tl, Bi, Ir, Rh, Fe vorhanden sein.

Ferner kann die Fällung auch in Gegenwart von Sensibilisierungsfarbstoffen erfolgen. Komplexierungsmittel und/oder Farbstoffe lassen sich zu jedem beliebigen Zeitpunkt unwirksam machen, z.B. durch Änderung des pH-Wertes oder durch eine oxidative Behandlung.

Nach abgeschlossener Kristallbildung oder auch schon zu einem früheren Zeitpunkt werden die löslichen Salze aus der Emulsion entfernt, z.B. durch Nudeln und Waschen, durch Flocken und Waschen, durch Ultrafiltration oder durch Ionenaustauscher.

Die Silberhalegenidemulsion wird im allgemeinen einer chemischen Sensibilisierung unter definierten Bedingungen - pH, pAg, Temperatur, Gelatine-, Silberhalogenid- und Sensibilisatorkonzentration - bis zum Erreichen des Empfindlichkeits- und Schleieroptimums unterworfen.

Die Verfahrensweise ist z.B. bei H. Frieser "Die Grundlagen der Photographischen Prozesse mit Silberhalogeniden" Seite 675-734, Akademische Verlagsgesellschaft (1968) beschrieben.

Dabei kann die chemische Sensibilisierung unter Zusatz von Verbindungen von Schwefel, Selen, Tellur und/oder Verbindungen der Metalle der VIII. Nebengruppe des Periodensystems (z.B. Gold, Platin, Palladium, Iridium) erfolgen, weiterhin können Thiocyanatverbindungen, oberflächenaktive Verbindungen, wie Thioether, heterocyclische Stickstoffverbindungen (z.B. Imidazole, Azaindene) oder auch spektrale Sensibilisatoren (beschrieben z.B. bei F. Hamer "The Cyanine Dyes and Related Compounds", 1964, bzw. Ullmanns Encyclopädie der technischen Chemie, 4. Auflage, Bd. 18, S. 431 ff. und Research Disclosure Nr. 17643, Abschnitt III) zugegeben werden. Ersatzweise oder zusätzlich kann eine Reduktionssensibilisierung unter Zugabe von Reduktionsmitteln (Zinn-II-Salze, Amine, Hydrazinderivate, Aminoborane, Silane, Formamidinsulfinsäure) durch Wasserstoff, durch niedrigen pAg (z.B. kleiner 5) und/oder hohen pH (z.B. über 8) durchgeführt werden.

Als Schwefelreifkörper kommen im allgemeinen zur Silbersulfidbildung befähigte Verbindungen z. B. Thiosulfat, Thioharnstoff, Thiosemicarbazid und Thiocarbamid in Frage. Thiosulfat ist bevorzugt.

Als Goldreifkörper werden anorganische Goldsalze eingesetzt. Aber auch organische Goldverbindungen wie in den Patenten DE-A-854 883 und DE-A-848 910 beschrieben, kommen in Frage.

Die Emulsionen können nur einer Schwefelreifung, aber auch einer kombinierten Schwefel-/Goldreifung (z. B. DE-A 22 63 910) unterworfen werden.

In einer weiteren bevorzugten Ausführungsform werden die Emulsionen iridiumdotiert, wobei die Iridiummenge 0,01 bis 0,5 µg/gAg beträgt.

Die fotografischen Emulsionen können Verbindungen zur Verhinderung der Schleierbildung oder zur Stabilisierung der fotografischen Funktion während der Produktion, der Lagerung oder der fotografischen Verarbeitung enthalten.

Besonders geeignet sind Azaindene, vorzugsweise Tetra-und Pentaazaindene, insbesondere solche, die mit Hydroxyl- oder Aminogruppen substituiert sind. Derartige Verbindungen sind z.B. von Birr, Z. Wiss. Phot. 47 (1952), S. 2-58 beschrieben worden. Weiter können als Antischleiermittel Salze von Metallen wie Quecksilber oder Cadmium, aromatische Sulfon- oder Sulfinsäuren wie Benzolsulfinsäure, oder stickstoffhaltige Heterocyclen wie Nitrobenzimidazol, Nitroindazol, (subst.) Benztriazole oder Benzthiazoliumsalze eingesetzt werden. Besonders geeignet sind Mercaptogruppen enthaltende Hetero cyclen, z.B. Mercaptobenzthiazole, Mercaptobenzimidazole, Mercaptotetrazole, Mercaptothiadiazole, Mercaptopyrimidine, wobei diese Mercaptoazole auch eine wasserlöslichmachende Gruppe, z.B. eine Carboxylgruppe oder Sulfogruppe, enthalten können. Weitere geeignete Verbindungen sind in Research Disclosure Nr. 17643 (1978), Abschnitt VI, veröffentlicht.

Die Stabilisatoren können den Silberhalogenidemulsionen vor, während oder nach deren Reifung zugesetzt werden. Selbstverständlich kann man die Verbindungen auch anderen fotografischen Schichten, die einer Halogensilberschicht zugeordnet sind, zusetzen.

Es können auch Mischungen aus zwei oder mehreren der genannten Verbindungen eingesetzt werden.

Die fotografischen Emulsionsschichten oder andere hydrophile Kolloidschichten des erfindungsgemäß hergestellten lichtempfindlichen Materials können oberflächenaktive Mittel für verschiedene Zwecke enthalten, wie Überzugshilfen, zur Verhinderung der elektrischen Aufladung, zur Verbesserung der Gleiteigenschaften, zum Emulgieren der Dispersion, zur Verhinderung der Adhäsion und zur Verbesserung der fotografischen Charakteristika (z.B. Entwicklungsbeschleunigung, hoher Kontrast, Sensibilisierung usw.).

Die fotografischen Emulsionen können unter Verwendung von Methinfarbstoffen oder anderen Farbstoffen spektral sensibilisiert werden. Besonders geeignete Farbstoffe sind Cyaninfarbstoffe, Merocyaninfarbstoffe und komplexe Merocyaninfarbstoffe.

Emulsionsherstellung

Nachfolgend wird die Herstellung einer monodispersen Silberchloridemulsion mit einer Korngröße von 0,8 µm beschrieben, wobei in einem Doppeleinlaufverfahren folgende Lösungen verwendet werden:

| Lösung E 1: | |
|---|---|
| Destilliertes Wasser<br>Gelatine | 1.000 ml<br>100 g |
| Lösung E 2: | |
| Destilliertes Wasser<br>Silbernitrat | 3.000 ml<br>1.000 g |
| Lösung E 3: | |
| Destilliertes Wasser<br>Ammoniumchlorid<br>$Na_2IrCl_6$-Lösung (0,01 %ig)<br>$Na_3RhCl_6$-Lösung (0,001 %ig) | 1.000 ml<br>370 g<br>5 g<br>1 ml |
| Lösung E 1: | |
| Destilliertes Wasser<br>Gelatine | 9.000 ml<br>900 g |

Die Vorlagelösung E 1 wurde auf 55 °C erwärmt und auf pH 5,0 eingestellt. Unter intensiver Durchmischung wurden die auf 55 °C erwärmten Lösungen E 2 und E 3 gleichzeitig innerhalb von 60 min. zudosiert. Zu Beginn betrug die Einlaufgeschwindigkeit 70 ml/min. Im weiteren Verlauf wurde langsam bis auf die 8fache Dosiergeschwindigkeit gesteigert. Nach Einlaufende wurde die Emulsion gekühlt und anschließend in üblicher Weise durch Flocken und Waschen von den löslichen Salzen befreit. Das gewaschene Flockulat wurde dann in der Lösung E 4 innerhalb von 30 min unter gleichzeitigem Rühren bei 40 °C redispergiert. Die Emulsion wurde anschließend mit 0,05 Mol-% KI und 0,4 Mol-% KBr pro Mol $AgNO_3$ versetzt und 120 min bei 50 °C und $2 \times 10^{-5}$ Mol Thiosulfat pro Mol $AgNO_3$ und $2 \times 10^{-6}$ Mol $HAuCl_4$ pro Mol $AgNO_3$ gereift. Nach Beendigung der Reifung wurde die Emulsion für den blauen Spektralbereich sensibilisiert (Sensibilisatormenge: $4 \times 10^{-4}$ Mol pro Mol $AgNO_3$) und mit 100 ml einer 1 %igen Lösung eines Azoindenstabilisators versetzt.

Zur sensitometrischen Prüfung wurde die Emulsion auf einer Papierunterlage gemeinsam mit einem Gelbkuppler gemäß der allgemeinen Formel I vergossen und nach Belichtung hinter einem Graukeil in einem Schnellverarbeitungsprozeß der im folgenden beschriebenen Ausführungsform verarbeitet.

Bei den erfindungsgemäßen farbfotografischen Aufzeichnungsmaterialien sind mindestens je eine überwiegend blauempfindliche, eine überwiegend grünempfindliche und eine überwiegend rotempfindliche Silberhalogenidemulsionsschicht übereinander auf einem Schichtträger aufgeschichtet. Falls die blauempfindliche Schicht in dem Schichtverband zuoberst angeordnet ist, kann sich unter ihr und oberhalb der grün- bzw. rotempfindlichen Schichten eine Schicht mit einem gelben Filterfarbstoff befinden. Desweiteren können zwischen zwei Silberhalogenidemulsionsschichten unterschiedlicher Spektralempfindlichkeit Zwischenschichten angeordnet sein. Die überwiegend blauempfindliche Silberhalogenidemulsionsschicht enthält einen Gelbkuppler, die überwiegend grünempfindliche Silberhalogenidemulsionsschicht einen Purpurkuppler und die überwiegend rotempfindliche Silberhalogenidemulsionsschicht einen Blaugrünkuppler. Bei dem erfindungsgemäßen Aufzeichnungsmaterial sind die lichtempfindlichen Schichten auf einem opaken

lichtreflektierenden Schichtträger aufgeschichtet, z.B. auf einem Schichtträger aus Papier, der eine Barytschicht tragen kann und/oder ein- oder beidseitig mit einer Schicht aus einem Polyolefin überschichtet sein kann.

Das fotografische Aufzeichnungsmaterial der vorliegenden Erfindung kann als Bindemittel für das Silberhalogenid und die Farbkuppler ein oder mehrere Polymere enthalten. Ein gebräuchliches Bindemittel ist Gelatine. Diese kann jedoch ganz oder teilweise durch andere synthetische, halbsynthetische oder auch natürlich vorkommende Polymere ersetzt werden. Synthetische Gelatineersatzstoffe sind beispielsweise Polyvinylalkohol, Poly-N-vinylpyrrolidon, Polyacrylamide, Polyacrylsäure und deren Derivate, insbesondere Mischpolymerisate. Natürlich vorkommende Gelatineersatzstoffe sind beispielsweise andere Proteine wie Albumin oder Casein, Cellulose, Zucker, Stärke oder Alginate. Halbsynthetische Gelatineersatzstoffe sind in der Regel modifizierte Naturprodukte. Cellulosederivate wie Hydroxyalkylcellulose, Carboxymethylcellulose und Phthalylcellulose sowie Gelatinederivate, die durch Umsetzung mit Alkylierungs- oder Acylierungsmitteln oder durch Aufpfropfung von polymerisierbaren Monomeren erhalten worden sind, sind Beispiele hierfür. Für die Verwendung als Bindemittel ist es von Bedeutung, daß die betreffenden Polymere noch über eine ausreichende Menge an funktionellen Gruppen verfügen, so daß durch Umsetzung mit geeigneten Härtungsmitteln genügend widerstandsfähige Schichten erzeugt werden können. Solche funktionellen Gruppen sind insbesondere Aminogruppen, aber auch Carboxylgruppen, Hydroxylgruppen und aktive Methylengruppen. Bevorzugtes Bindemittel des erfindungsgemäßen farbfotografischen Aufzeichnungsmaterials ist Gelatine.

Die Bindemittelschichten des erfindungsgemäßen Aufzeichnungsmaterials sind mit einem Härtungsmittel der Formel II gehärtet. Solche Härtungsmittel sind beispielsweise in DE-A-24 39 551 beschrieben. Beispiele für solche Härtungsmittel (H-) sind im folgenden aufgeführt:

H-1 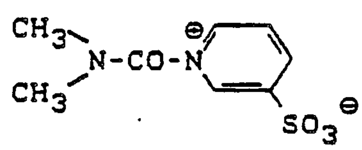

H-2 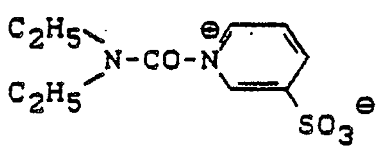

H-3

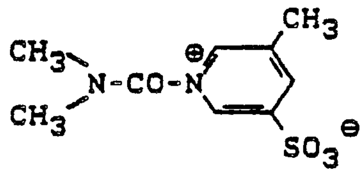

H-4

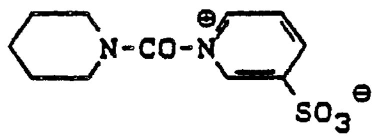

H-5

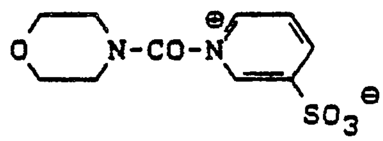

H-6

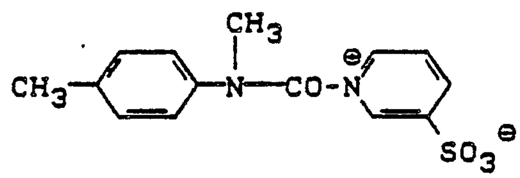

H-7

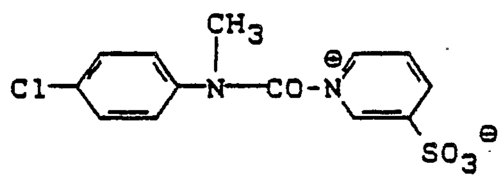

H-8

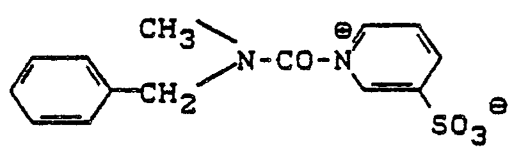

H-9

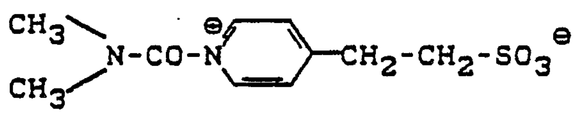

H-10

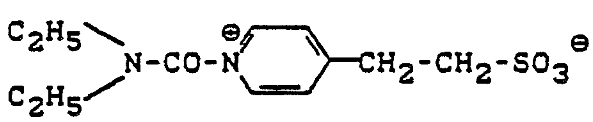

H-11 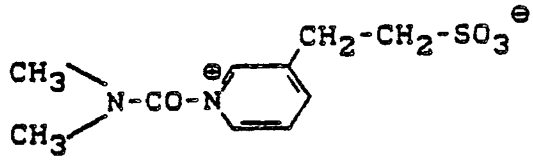

H-12 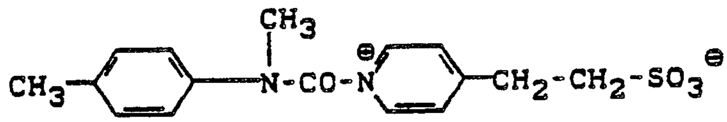

H-13 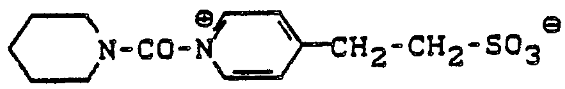

H-14 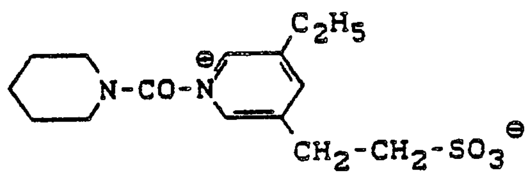

H-15 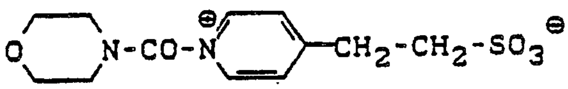

H-16 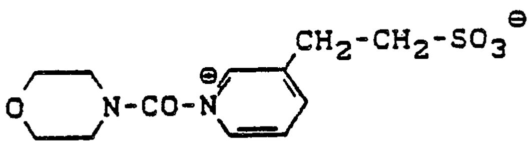

H-17 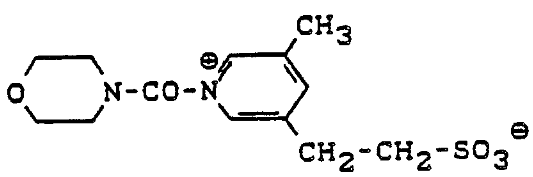

H-18 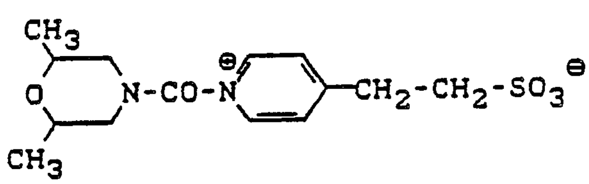

H-19

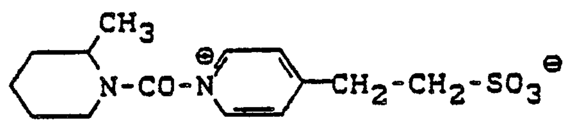

H-20

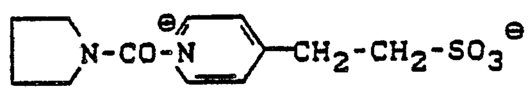

Bei der Herstellung des lichtempfindlichen farbfotografischen Aufzeichnungsmaterials können die diffusionsfesten Kuppler der vorliegenden Erfindung in bekannter Weise in die Gießlösung der Silberhalogenidemulsionsschichten oder anderer Kolloidschichten eingearbeitet werden. Beispielsweise können die öllöslichen oder hydrophoben Kuppler vorzugsweise aus einer Lösung in einem geeigneten Kupplerlösungsmittel (Ölbildner) gegebenenfalls in Anwesenheit eines Netz- oder Dispergiermittels zu einer hydrophilen Kolloidlösung zugefügt werden. Die hydrophile Gießlösung kann selbstverständlich neben dem Bindemittel andere übliche Zusätze enthalten. Die Lösung des Kupplers braucht nicht direkt in die Gießlösung für die Silberhalogenidemulsionsschicht oder eine andere wasserdurchlässige Schicht dispergiert zu werden; sie kann vielmehr auch vorteilhaft zuerst in einer wäßrigen nichtlichtempfindlichen Lösung eines hydrophilen Kolloids dispergiert werden, worauf das erhaltene Gemisch gegebenenfalls nach Entfernung der verwendeten niedrig siedenden organischen Lösungsmittel mit der Gießlösung für die lichtempfindliche Silberhalogenidemulsionsschicht oder einer anderen wasserdurchlässigen Schicht vor dem Auftragen vermischt wird.

Entsprechende Methoden sind beispielsweise beschrieben in US-A-2 322 027, DE-A-1 722 192 und EP-A-0 043 037. Die Verbindungen können auch in Form beladener Latices in die Gießlösung eingebracht werden. Verwiesen wird beispielsweise auf DE-A-25 41 230, DE-A-25 41 274, DE-A-28 35 856, EP-A-0 014 921, EP-A-0 069 671, EP-A-0 130 115, US-A-4 291 113, US-A-4 388 403.

Das erfindungsgemäße farbfotografische Aufzeichnungsmaterial zur Herstellung mehrfarbiger Bilder enthält in räumlicher und spektraler Zuordnung zu den Silberhalogenidemulsionsschichten unterschiedlicher Spektralempfindlichkeit Farbkuppler zur Erzeugung der unterschiedlichen Teilfarbenbilder Cyan, Purpur und Gelb. Unter räumlicher Zuordnung ist dabei zu verstehen, daß der Farbkuppler sich in einer solchen räumlichen Beziehung zu der Silberhalogenidemulsionsschicht befindet, daß eine Wechselwirkung zwischen ihnen möglich ist, die eine bildgemäße Übereinstimmung zwischen dem bei der Entwicklung gebildeten Silberbild und dem aus dem Farbkuppler erzeugten Farbbild zuläßt. Dies wird in der Regel dadurch erreicht, daß der Farbkuppler in der Silberhalogenidemulsionsschicht selbst enthalten ist oder in einer hierzu benachbarten gegebenenfalls nichtlichtempfindlichen Bindemittelschicht.

Unter spektraler Zuordnung ist zu verstehen, daß die Spektralempfindlichkeit jeder der lichtempfindlichen Silberhalogenidemulsionsschichten und die Farbe des aus dem jeweils räumlich zugeordneten Farbkuppler erzeugten Teilfarbenbildes in einer bestimmten Beziehung zueinander stehen, wobei jeder der Spektralempfindlichkeiten (Rot, Grün, Blau) eine andere Farbe des betreffenden Teilfarbenbildes (im allgemeinen z.B. die Farben Cyan, Purpur bzw. Gelb in dieser Reihenfolge) zugeordnet ist.

Jeder der unterschiedlich spektral sensibilisierten Silberhalogenidemulsionsschichten kann ein oder können auch mehrere Farbkuppler zugeordnet sein. Wenn mehrere Silberhalogenidemulsionsschichten gleicher Spektralempfindlichkeit vorhanden sind, kann jede von ihnen einen Farbkuppler enthalten, wobei diese Farbkuppler nicht notwendigerweise identisch zu sein brauchen. Sie sollen lediglich bei der Farbentwicklung wenigstens annähernd die gleiche Farbe ergeben, normalerweise eine Farbe, die komplementär ist zu der Farbe des Lichtes, für das die betreffenden Silberhalogenidemulsionsschichten überwiegend empfindlich sind.

Rotempfindlichen Silberhalogenidemulsionsschichten ist folglich bei bevorzugten Ausführungsformen mindestens ein nichtdiffundierender Farbkuppler zur Erzeugung des blaugrünen Teilfarbenbildes zugeordnet, in der Regel ein Kuppler vom Phenol- oder α-Naphtholtyp. Geeignete Farbkuppler sind in EP-A-0 184 057, EP-A-0 175 573, EP-A-0 161 626, EP-A-0 028 099, EP-A-0 067 689, EP-A-0 142 086, DE-A-2 028 601 beschrieben.

Grünempfindlichen Silberhalogenidemulsionsschichten ist mindestens ein nichtdiffundierender Farbkuppler zur Erzeugung des purpurnen Teilfarbenbildes zugeordnet, wobei üblicherweise Farbkuppler vom Typ des 5-Pyrazolons, des Indazolons oder verschiedener Pyrazoloazole Verwendung finden. Purpurkuppler dieser Art sind in DE-A-3 516 996 und DE-A-3 516 945 zu finden.

Blauempfindlichen Silberhalogenidemulsionsschichten schließlich ist mindestens ein nichtdiffundierender Farbkuppler zur Erzeugung des gelben Teilfarbenbildes zugeordnet, im vorliegenden Fall ein Gelbkuppler der allgemeinen Formel I. Farbkuppler dieser Art sind in großer Zahl bekannt und in einer Vielzahl von Patentschriften beschrieben. Beispielhaft sei hier auf die Veröffentlichungen "Farbkuppler" von W. PELZ in "Mitteilungen aus den Forschungslaboratorien der Agfa, Leverkusen/München", Band III, Seite 111 (1961) und von K. VENKATARAMAN in "The Chemistry of Synthetic Dyes", Vol. 4, 341 bis 387, Academic Press (1972), verwiesen.

Bei den Farbkupplern kann es sich sowohl um übliche 4-Äquivalentkuppler handeln als auch um 2-Äquivalentkuppler, bei denen zur Farberzeugung eine geringere Menge Silberhalogenid erforderlich ist. 2-Äquivalentkuppler leiten sich bekanntlich von den 4-Äquivalentkupplern dadurch ab, daß sie in der Kupplungsstelle einen Substituenten enthalten, der bei der Kupplung abgespalten wird. Zu den 2-Äquivalentkupplern sind sowohl solche zu rechnen, die praktisch farblos sind, als auch solche, die eine intensive Eigenfarbe aufweisen, die bei der Farbkupplung verschwindet bzw. durch die Farbe des erzeugten Bildfarbstoffes ersetzt wird. Letztere Kuppler können ebenfalls zusätzlich in den lichtempfindlichen Silberhalogenidemulsionsschichten vorhanden sein und dort als Maskenkuppler zur Kompensierung der unerwünschten Nebendichten der Bildfarbstoffe dienen. Zu den 2-Äquivalentkupplern sind aber auch die bekannten Weißkuppler zu rechnen, die jedoch bei Reaktion mit Farbentwickleroxidationsprodukten keinen Farbstoff ergeben. Zu den 2-Äquivalentkupplern sind ferner die bekannten DIR-Kuppler zu rechnen, bei denen es sich um Kuppler handelt, die in der Kupplungsstelle einen abspaltbaren Rest enthalten, der bei Reaktion mit Farbentwickleroxidationsprodukten als diffundierender Entwicklungsinhibitor in Freiheit gesetzt wird. Auch andere fotografisch wirksame Verbindungen, z. B. Entwicklungsaceferatoren oder Schleiermittel, können bei der Entwicklung aus solchen Kupplern freigesetzt werden.

Die Kuppler einschließlich der erfindungsgemäß verwendeten Verbindungen der Formel I können auch in polymerer Form, z.B. als Polymerisatlatex zur Anwendung gelangen.

Hochmolekulare Farbkuppler sind beispielsweise beschrieben in DE-C-1 297 417, DE-A-24 07 569, DE-A-31 48 125, DE-A-32 17 200, DE-A-33 20 079, DE-A-33 24 932, DE-A-33 31 743, DE-A-33 40 376, EP-A-27 284, US-A-4 080 211. Die hochmolekularen Farbkuppler werden in der Regel durch Polymerisation von ethylenisch ungesättigten monomeren Farbkupplern hergestellt.

Die verwendeten Farbkuppler können auch solche sein, die Farbstoffe mit einer schwachen bzw. eingeschränkten Beweglichkeit liefern.

Über die genannten Bestandteile hinaus kann das farbfotografische Aufzeichnungsmaterial der vorliegenden Erfindung weitere Zusätze enthalten, wie zum Beispiel Antioxidantien, farbstoffstabilisierende Mittel und Mittel zur Beeinflussung der mechanischen und elektrostatischen Eigenschaften. Um die nachteilige Einwirkung von UV-Licht auf die mit dem erfindungsgemäßen farbfotografischen Aufzeichnungsmaterial hergestellten Farbbilder zu vermindern oder zu vermeiden, ist es beispielsweise vorteilhaft, in einer oder mehreren der in dem Aufzeichnungsmaterial enthaltenen Schichten, vorzugsweise in einer der oberen Schichten, UV-absorbierende Verbindungen zu verwenden.

Geeignete UV-Absorber sind beispielsweise in US-A-3 253 921, DE-C-2 036 719 und EP-A-0 057 160 beschrieben.

Zur Herstellung farbfotografischer Bilder wird das erfindungsgemäße farbfotografische Aufzeichnungsmaterial mit einer Farbentwicklerverbindung entwickelt. Als Farbentwicklerverbindung lassen sich sämtliche Entwicklerverbindungen verwenden, die die Fähigkeit besitzen, in Form ihres Oxidationsproduktes mit Farbkupplern zu Azomethinfarbstoffen zu reagieren. Geeignete Farbentwicklerverbindungen sind aromatische mindestens eine primäre Aminogruppe enthaltende Verbindungen vom p-Phenylendiamintyp, beispielsweise N,N-Dialkyl-p-phenylendiamine, wie N,N-Diethyl-p-phenylendiamin, 1-(N-ethyl-N-methylsulfonamidoethyl)-3-methyl-p-phenylendiamin, 1-(N-ethyl-N-hydroxyethyl-3-methyl-p-phenylendiamin und 1-(N-ethyl-N-methoxyethyl)-3-methyl-p-phenylendiamin.

Weitere brauchbare Farbentwickler sind beispielsweise beschrieben in J. Amer. Chem. Soc. 73, 3100 (1951) und in G. Haist, Modern Photographic Processing, 1979, John Wiley and Sons, New York, Seiten 545 ff.

Das erfindungsgemäße Aufzeichnungsmaterial eignet sich vorzüglich für die Verarbeitung in einem abgekürzten Verarbeitungsprozeß, z.B. in einem Verarbeitungsprozeß, dessen Entwicklungsschritt bei Temperaturen zwischen 30 und 80°C weniger als 3 Minuten, vorzugsweise weniger als 1 Minute dauert. Speziell bei Entwicklung mit benzylalkoholfreien Entwicklerbädern werden vorteilhafte Ergebnisse erhalten.

Nach der Farbentwicklung wird das Material üblicherweise gebleicht und fixiert. Bleichung und Fixierung können getrennt voneinander oder auch zusammen durchgeführt werden. Als Bleichmittel können die üblichen Verbindungen verwendet werden, z.B. $Fe^{3+}$-Salze und $Fe^{3+}$-Komplexsalze wie Ferricyanide, Dichromate, wasserlösliche Kobaltkomplexe usw. Besonders bevorzugt sind Eisen-III-Komplexe von Aminopolycarbonsäuren insbe sondere z.B. Ethylendiamintetraessigsäure, N-Hydroxyethylethylendiamintriessigsäure, Alkyliminodicarbonsäuren und von entsprechenden Phosphonsäuren. Geeignet als Bleichmittel sind

weiterhin Persulfate.

Im folgenden sind die Zusammensetzungen und Verarbeitungszeiten von Entwicklungs- und Bleichfixierbad angegeben, in denen das erfindungsgemäße Material verarbeitet wird:

| | |
|---|---|
| a) Farbentwickler - 45 s - 35 °C | |
| Triethanolamin | 9,0 g/l |
| NN-Diethylhydroxylamin | 4,0 g/l |
| Diethylenglykol | 0,05 g/l |
| 3-Methyl-4-amino-N-ethyl-N-methansulfonamidoethyl-anilin-sulfat | 5,0 g/l |
| Kaliumsulfit | 0,2 g/l |
| Triethylenglykol | 0,05 g/l |
| Kaliumcarbonat | 22 g/l |
| Kaliumhydroxid | 0,4 g/l |
| Ethylendiamintetraessigsäure di-Na-Salz | 2,2 g/l |
| Kaliumchlorid | 2,5 g/l |
| 1,2-Dihydroxybenzol-3,4,6-trisulfonsäure-trinatriumsalz | 0,3 g/l |
| auffüllen mit Wasser auf 1.000 ml; pH 10,0 | |
| b) Bleichfixierbad - 45 s - 35 °C | |
| Ammoniumthiosulfat | 75 g/l |
| Natriumhydrogensulfit | 13,5 g/l |
| Ammoniumacetat | 2,0 g/l |
| Ethylendiamintetraessigsäure (Eisen-Ammonium-Salz) | 57 g/l |
| Ammoniak 25 %ig | 9,5 g/l |
| Essigsäure | 9,0 g/l |
| auffüllen mit Wasser auf 1.000 ml; pH 5,5 | |
| c) Wässern - 2 min - 33 °C | |

Beispiel

Ein farbfotografisches Aufzeichnungsmaterial, welches für einen Schnellverarbeitungsprozeß geeignet ist, wurde hergestellt, indem auf einen Schichtträger auf beidseitig mit Polyethylen beschichtetem Papier die folgenden Schichten in der angegebenen Reihenfolge aufgetragen wurden. Die Mengenangaben beziehen sich jeweils auf 1 $m^2$. Für den Silberhalogenidauftrag werden die entsprechenden Mengen $AgNO_3$ angegeben.

Schichtaufbau 1:

1. Schicht (Substratschicht):

0,2 g Gelatine

2. Schicht (blauempfindliche Schicht):

blauempfindliche Silberhalogenidemulsion (99,5 Mol-% Chlorid, 0,5 Mol-% Bromid, mittlerer Korndurchmesser 0,8 µm) aus 0,63 g $AgNO_3$ mit

1,38 g Gelatine

0,95 g Gelbkuppler Y-10

0,2 g Weißkuppler W-1

0,29 g Trikresylphosphat (TKP)

3. Schicht (Schutzschicht)

1,1 g Gelatine

0,06 g 2,5-Dioctylhydrochinon

0,06 g Dibutylphthalat (DBP)

4. Schicht (grünempfindliche Schicht)

grünsensibilisierte Silberhalogenidemulsion (99,5 Mol-% Chlorid, 0,5 Mol-% Bromid, mittlerer Korndurchmesser 0,6 µm) aus 0,45 g $AgNO_3$ mit 1,08 g Gelatine

0,41 g Purpurkuppler M-1

0,16 g α-(3-t-Butyl-4-hydroxyphenoxy)-myristinsäureethylester

0,08 g 2,5-Dioctylhydrochinon

0,34 g DBP

0,04 g TKP

5. Schicht (UV-Schutzschicht)

1,15 g Gelatine

0,6 g UV-Absorber Tinuvin 343®

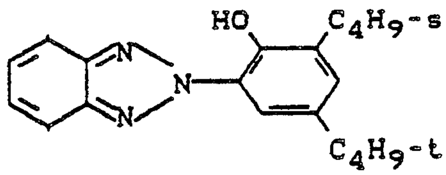

0,045 g 2,5-Dioctylhydrochinon

0,04 g TKP

6. Schicht (rotempfindliche Schicht)

rotsensibilisierte Silberhalogenidemulsion (99,5 Mol-% Chlorid, 0,5 Mol-% Bromid, mittlerer Korndurchmesser 0,5 µm) aus 0,3 g $AgNO_3$ mit

0,75 g Gelatine

0,36 g Blaugrünkuppler C-1

0,36 g TKP

7. Schicht (UV-Schutzschicht)

0,35 g Gelatine

0,15 g UV-Absorber Tinuvin 343®

0,2 g TKP

8. Schicht (Schutzschicht)

0,9 g Gelatine

0,3 g Härtungsmittel H-15 der folgenden Formel

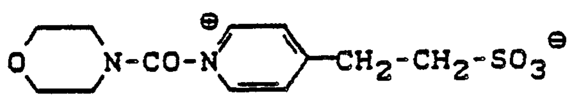

Schichtaufbau 2:

wie Schichtaufbau 1, jedoch mit
1 g Gelbkuppler Y-1 in Schicht 2.

Schichtaufbau 3:

wie Schichtaufbau 1, jedoch mit
1,03 g Gelbkuppler Y-2 in Schicht 2.

Schichtaufbauten 4 - 6:

wie in den Schichtaufbauten 1 - 3, jedoch mit
0,35 g Härtungsmittel H-21 der folgenden Formel

$CH_2=CH-SO_2-CH_2-SO_2-CH=CH_2$ H-21

Die Schichtaufbauten 1 - 3 stellen die erfindungsgemäßen Materialien dar, während die Aufbauten 4 - 6 als Vergleich dienen.

Als Farbkuppler wurden folgende Verbindungen verwendet:

Y-1

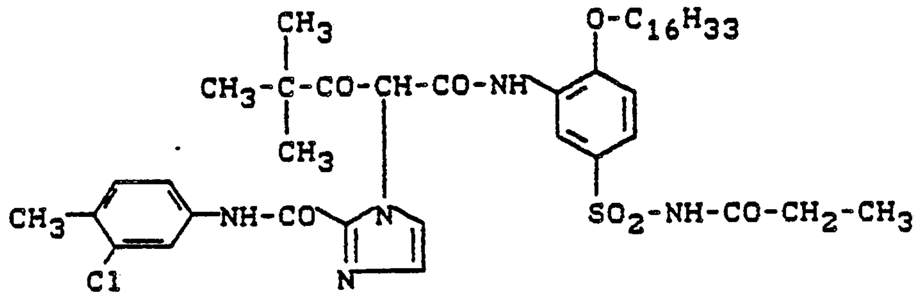

Y-2

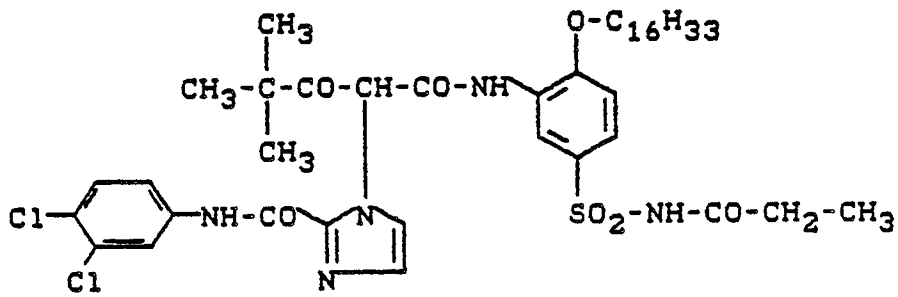

Y-10

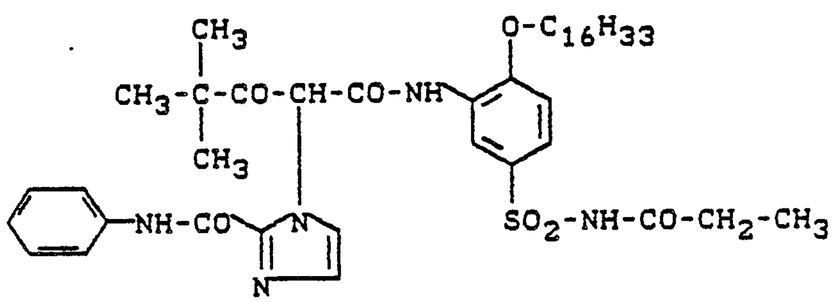

M-1

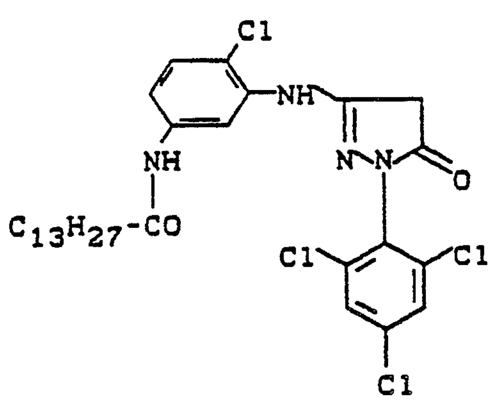

C-1

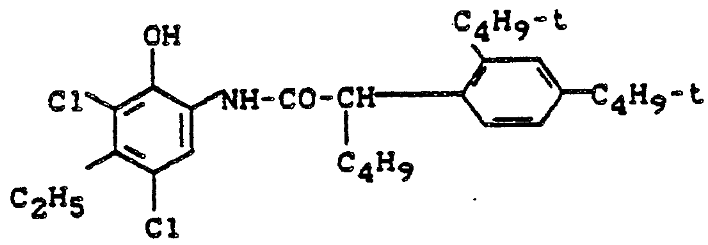

W-1

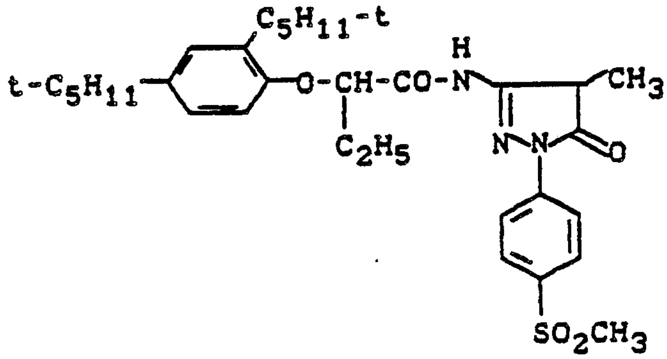

Die Schichtaufbauten wurden hinter einem Graukeil belichtet und einem Schnellverarbeitungsprozeß gemäß den auf Seite 36 genannten Bedingungen unterworfen. Anschließend wurde die Gelbminimaldichte (Gelbschleier) hinter einem Blaufilter gemessen und ist für die Aufbauten 1 - 6 in der folgenden Tabelle ersichtlich:

Tabelle 1:

| Schichtaufbau | minimale Farbdichte $D_{min}$ |
|---|---|
| 1 (erfindungsgemäß) | 0,176 |
| 2 " | 0,177 |
| 3 " | 0,165 |
| 4 (Vergleich) | 0,188 |
| 5 " | 0,179 |
| 6 " | 0,176 |

Bei Vergleich der Schichtaufbauten 1 mit 4, 2 mit 5 und 3 mit 6 wird offensichtlich, daß die erfindungsgemäßen Aufbauten 1,2 und 3, die mit dem erfindungsgemäßen Härtungsmittel H-15 gehärtet worden sind, im Gegensatz zu den Vergleichsaufbauten 4, 5 und 6, bei denen das Härtungsmittel H-21 verwendet wurde, einen deutlich niedrigeren Gelbschleier unter den Bedingungen der Schnellverarbeitung aufweisen.

## Ansprüche

1. Farbfotografisches Aufzeichnungsmaterial, das auf einem reflektierenden Schichtträger mindestens eine blauempfindliche Silberhalogenidemulsionsschicht und einen dieser zugeordneten Gelbkuppler, mindestens eine grünempfindliche Silberhalogenidemulsionsschicht und einen dieser zugeordneten Purpurkuppler, mindestens eine rotempfindliche Silberhalogenidemulsionsschicht und einen dieser zugeordneten Blaugrünkuppler und gegebenenfalls weitere nicht lichtempfindliche Schichten enthält, dadurch gekennzeichnet, daß

a) die Silberhalogenidemulsionsschichten einen Chloridgehalt von ≧ 95 Mol-% aufweisen,

b) der blauempfindlichen Silberhalogenidemulsionsschicht ein nicht diffundierender α-Acylacetanilid-Gelbkuppler der folgenden Formel (I) zugeordnet ist:

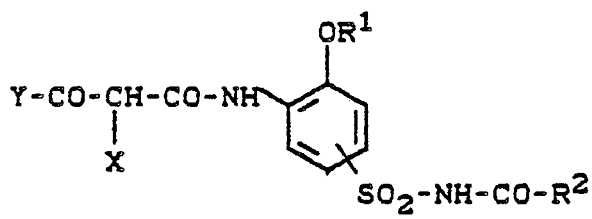

(I)

worin bedeuten:
Y einen aliphatischen oder cycloaliphatischen Rest;
X ein Wasserstoffatom oder eine bei Farbkupplung abspaltbare Gruppe;
$R^1$ einen Alkylrest mit 12 bis 20 C-Atomen oder eine Bindung an ein Polymergerüst;
$R^2$ einen Alkylrest mit 1 bis 4 C-Atomen oder eine Bindung an ein Polymergerüst:

c) die Schichten des Aufzeichnungsmaterials mit einem Härtungsmittel der folgenden Formel (II) gehärtet sind

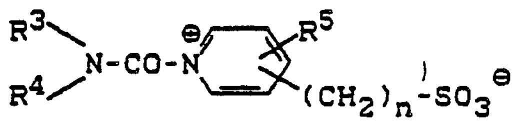

(II)

worin bedeuten:
$R^3$ und $R^4$ einzeln gleich oder verschieden, jeweils eine Alkylgruppe mit 1 bis 8 Kohlenstoffatomen oder eine gegebenenfalls mit einer Alkylgruppe mit 1 oder 2 Kohlenstoffatomen oder mit einem Halogenatom substituierte Aryl- oder Aralkylgruppe, oder zusammen die zur Vervollständigung eines gegebenenfalls mit einer Alkylgruppe mit 1 oder 2 Kohlenstoffatomen oder mit einem Halogenatom substituierten heterocyclischen Ringes, z. B. eines Piperidin- oder Morpholinringes erforderlichen Atome,
$R^5$ ein Wasserstoffatom oder eine Alkylgruppe mit 1 oder 2 Kohlenstoffatomen,
n gleich 0 oder 2.

2. Farbfotografisches Aufzeichnungsmaterial nach Anspruch 1, dadurch gekennzeichnet, daß die Silberhalogenidemulsion 0 bis 5 Mol-% Bromid, Iodid und/oder Rhodanid enthalten.

3. Farbfotografisches Aufzeichnungsmaterial nach Anspruch 1, dadurch gekennzeichnet, daß die Silberhalogenidemulsionen einen Bromidgehalt von 0,02 bis 5 Mol-% aufweisen.

4. Farbfotografisches Aufzeichnungsmaterial nach Anspruch 1, dadurch gekennzeichnet, daß Y in Formel (I) einen tertiären Butylrest bedeutet.